# EUROPEAN PATENT APPLICATION

(11) **EP 3 560 518 A2**
(43) Date of publication of application: **30.10.2019**
(21) Application number: 17888910.1
(22) Date of filing: 13.12.2017
(51) Int. Cl.: A61K 47/54, A61K 47/64, A61K 9/70, A61K 31/7088, A61K 38/00, A61K 38/43

(54) **COMPLEX FOR DRUG DELIVERY AND STABILIZATION AND PREPARATION METHOD THEREOF**

(30) Priority: 26.12.2016 KR 20160179122
(71) Applicant: Interoligo Corporation, Gyeonggi-do 16006 (KR)
(72) Inventor: LEE, Jung Hwan, Seoul 06558 (KR); LIM, Jong Hoon, Seoul 05501 (KR); KIM, Jong In, Seoul 07226 (KR); LEE, Kyoung Ho, Seoul 07249 (KR); KIM, Yoo Jin, Seoul 07741 (KR); LEE, Jong Wook, Seongnam-si Gyeonggi-do 13464 (KR); CHOI, Ji Ah, Namyangju-si Gyeonggi-do 12251 (KR)
(74) Representative: dompatent von Kreisler Selting Werner - Partnerschaft von Patent- und Rechtsanwälten mbB
(86) International application number: PCT/KR2017/014593
(87) International publication number: WO 2018/124548

(57) **Abstract**

The present invention provides an anticancer composition that can compensate for shortcomings of existing anticancer agents and anticancer therapies, and a formulation thereof, wherein the anticancer composition includes an atelocollagen-[aptamer-X] complex. In the above formula, X is at least one selected from the group consisting of drugs, toxins, oligonucleotide drugs (siRNA, miRNA, antisense, etc.), proteins (antibodies), enzymes and nanoparticles.

SEQUENCE LISTING

<110>  INTEROLIGO CORPORATION

<120>  Complex For Stabilizing And Delivery Of Drugs And Processes For
       Preparing The Same

<130>  05008

<140>  PCT/KR2017/014593
<141>  2017-12-13

<150>  KR 10-2016-0179122
<151>  2016-12-26

<160>  5

<170>  PatentIn version 3.5

<210>  1
<211>  26
<212>  DNA
<213>  Artificial Sequence

<220>
<223>  AS1411 aptamer

<400>  1
ggtggtggtg gttgtggtgg tggtgg                                          26


<210>  2
<211>  26
<212>  DNA
<213>  Artificial Sequence

<220>
<223>  CRO aptamer

<400>  2
cctcctcctc cttctcctcc tcctcc                                          26


<210>  3
<211>  40
<212>  DNA
<213>  Artificial Sequence

<220>
<223>  ERBB2, 6 = NapdU [5-(N-naphthylcarboxyamide)-2'-deoxyuridine]

<400>  3
asgssagags ssgccsgags gccscgcaag ggcgsaacaa                          40


<210>  4
<211>  29
<212>  DNA
<213>  Artificial Sequence

<220>
<223>  T3-AS1411

<400>  4
tttggtggtg gtggttgtgg tggtggtgg                                          29

<210>  5
<211>  29
<212>  DNA
<213>  Artificial Sequence

<220>
<223>  T3-CRO

<400>  5
tttcctcctc ctccttctcc tcctcctcc                                          29

## Description

### [Technical Field]

The present invention relates to a complex for drug delivery and stabilization and a preparation method thereof, and more particularly, to a complex for stabilizing an aptamer-L-X conjugate [Aptameur-Drug conjugate] which is prepared by attaching a therapeutic material (X) (with therapeutic effects) to an aptamer using a linker (L) in order to utilize a targeting ability of the aptamer, thereby protecting the aptamer in a body for a long time, and a preparation method thereof.

### [Background Art]

Pancreatic cancer is a desperate cancer without a significant change in a survival rate since any distinct treatment method has not come out for decades. The reason for this is believed that the pancreatic cancer is a notorious cancer with very little response to traditional anticancer chemicals or new formulations of immune anticancer drug chemicals, and fibrous tissues surrounding tumors as a typical feature of the pancreatic cancer interfere with immune therapeutic response. 85% of patients are found at the last stage and involve high recurrence rate, and 15 new patients with pancreatic cancer occur and 14 patients die every day.

The basic chemotherapy for pancreatic cancer is currently to use gemcitabine alone. Till now, any existing chemotherapy selected for treatment can only ensure an average survival time of 8 to 12 months for locally advanced pancreatic cancer, and 3 to 6 months for metastatic pancreatic cancer. Treatment of pancreatic cancer has been performed by a combination therapy of gemcitabine and a number of drugs over the past 12 years, however, achieved barely non-significant benefit, as compared to using gemcitabine alone. However, the combination therapy of two or three types of drugs while gemcitabine has a primary role is still executed. In this regard, it is a question of whether the therapy should depend on only gemcitabine or a combination therapy involving alternative agents should be performed. Further, although it seems to step forward with introduction of various targeted therapeutic agents in accordance with identification of a molecular biological carcinogenesis process, effects thereof are substantially not prominent. For such reasons, development of biological targeted therapeutic agents and novel cytotoxic drugs is urgently required more than ever. Further, discovery of new drugs and development of combination therapies for clinical trials should be continued, and a treatment method for alleviating severe pain and cachexia will also be required.

Breast cancer is the most common cancer among female cancers in developed counties including the United States and Europe, etc., and is the first major cause of death in American women in 40 to 55 year-old ages. The breast cancer occurs in one of 9 women for a lifetime and has a tendency to increase in the number of patients by about 15% annually. In Korea, breast cancer occupied about 11.9% of female cancer patients in 1995, has been the third common cancer following cervical cancer and stomach cancer, and showed with fifth high mortality following gastric cancer, liver cancer, uterine cancer and lung cancer, with its frequency tending to increase every year.

Liver cancer is a significant disease in socio-economic aspect at home and abroad. With regard to cancer mortality in Korea as of 2012, the liver cancer is the second common cause of cancer-related death, and 22.5 deaths per 100,000 population were due to liver cancer. In particular, Korea is known to have high incidence of liver cancer due to high prevalence rate of hepatitis type B. In addition, a loss of income due to liver cancer is known to cause the greatest socio-economic loss among diverse cancers, and therefore, there is an urgent requirement for development of effective therapeutic agents. Currently developed standard therapeutic agents include, for example, sorafenip, which is an anticancer targeted therapeutic agent for oral use and has been proved to inhibit different cellular materials, thus being effective in treatment of liver cancer. However, even if these therapeutic agents have therapeutic effects, they reached limitation since an increase in survival time is only 2 to 3 months.

In a case of colorectal cancer rapidly increasing due to a westernized diet, surgery or chemotherapy has been tried as a method for treatment of the colorectal cancer. 5-fluorouracil (5-FU) is a major anti-tumor compound selected for treatment of the colorectal cancer, but has been demonstrated to be effective in only limited range. 5-FU can temporarily decrease a size of the colorectal cancer, but has very few evidences indicating that survival of patients is substantially extended or 'healed' (based on 5 years relief period). The chemotherapy with 5-FU is used in patients with a disease spreading to the liver, however, a temporary improvement is observed in only 25% or less of such cases and there is no significant influence on overall survival rate.

Further, in a case of thyroid cancer particularly appearing to be rapidly increasing in Korean people in recent years, excessive surgery is being raised as a problem.

Drugs used in treatment for cancer mostly represent serious side effects or toxicity. Recently, aptamer specifically binding to a receptor associated with cancer has been developed, and the aptamer alone or in combination with other drugs is under clinical trials for treatment of various cancers including breast cancer. The present inventors have already developed an aptamer specifically binding to Her-2 receptor and disclosed the same in U. S. Patent Publication No. US9409515B2.

In particular, such an aptamer has a targeting function and, when the aptamer is conjugated with some materials X with anti-cancer effects, for example, drugs, toxins, oligonucleotide drugs (siRNA, miRNA, antisense, etc.), proteins, enzymes, nanoparticles, etc., the aptamer may deliver the above materials such as drugs to cancer cells of a target. However, such an aptamer-L-X conjugate (aptamer-drug conjugate) is unstable in the body, and cannot properly exhibit its original functions in many cases.

Similarly, other drugs such as arthritis medicines require a technology capable of stabilizing the drugs *in vivo* when conjugating the drugs with the aptamer.

### [Disclosure]

### [Technical Problem]

Accordingly, an object of the present invention is to provide a therapeutic agent and a formulation thereof that can compensate for shortcomings of existing drugs and treatment using the same.

In particular, another object of the present invention is to stabilize an aptamer-L-X conjugate (aptamer-drug conjugate) unstable *in vivo,* thereby attaining excellent therapeutic effects.

### [Technical Solution]

The present inventors found that, when an aptamer-L-X conjugate (e.g., aptamer-drug conjugate) is combined with atelocollagen to form an atelocollagen-(aptamer-L-X conjugate) complex and this complex is used, the aptamer-L-X conjugate may be stabilized *in vivo* so as to maximize effects thereof, and the present invention has been completed on the basis of the finding. That is, a novel atelocollagen-mediated local aptamer-linker-drug conjugate delivery system is provided.

In other words, the present invention relates to a drug-stabilizing complex including an atelocollagen-[aptamer-X] complex, wherein X is at least one selected from the group consisting of drugs, toxins, oligonucleotide drugs (siRNA, miRNA, antisense, etc.), proteins (antibodies), enzymes and nanoparticles.

### [Advantageous effects]

The complex of the present invention is stable *in vivo* and exhibits excellent therapeutic effects including anticancer effects.

### [Brief Description of Drawings]

FIG. 1 illustrates HPLC RT of AS1411-Dox adduct after reaction of AS1411 and doxorubicin according to an embodiment of the present invention.
FIG. 2 illustrates UV-visible absorption spectrum of AS1411-Dox adduct according to an embodiment of the present invention.
FIG. 3 illustrates ERBB2 aptamer-siRNA conjugate according to an embodiment of the present invention.
FIG. 4 is photographs showing an atelocollagen-[aptamer-drug conjugate] complex (sol-gel type) according to an embodiment of the present invention.
FIG. 5 is photographs showing atelocollagen-[aptamer-drug conjugate] complexes (patch type) according to an embodiment of the present invention.
FIG. 6 is electron micrographs showing an atelocollagen-[aptamer-drug conjugate] complex (scaffold type) according to an embodiment of the present invention.
FIG. 7 is photographs (FIG. 7A) and graphs (FIG. 7B) showing results of measuring *in vivo* stability of Cy3-AS1411-Gem conjugate and atelocollagen-[Cy3-AS1411-Gem conjugate] complex (sol-gel type).
FIG. 8 is a photograph showing atelocollagen-[aptamer-drug conjugate] complexes (patch type) according to an embodiment of the present invention.
FIG. 9 is photographs showing test results of *in vivo* stability of the atelocollagen-[aptamer-drug conjugate] complexes (patch type) according to the embodiment of the present invention.
FIG. 10 is a photograph (FIG. 10A) and electron micrographs (SEM images) (FIG. 10B) showing atelocollagen-[aptamer-drug conjugate] complexes (scaffold type) according to an embodiment of the present invention.
FIG. 11 is photographs showing test results of *in vivo* stability of the atelocollagen-[aptamer-drug conjugate] complexes (scaffold type) according to the embodiment of the present invention.
FIG. 12 is photographs (FIG. 12A) and electron micrographs (SEM images) (FIG. 12B) showing atelocollagen-[Cy3-AS1411-Gem conjugate] complex (scaffold type) according to an embodiment of the present invention.
FIG. 13 is graphs illustrating efficacy examination results of AS1411, CRO, gemcitabine, AS1411-Gem conjugate and CRO-Gem conjugate to pancreatic cancer cell-lines.
FIG. 14 is graphs illustrating efficacy examination results of AS1411, CRO, gemcitabine, atelocollagen-[AS1411-Gem conjugate] and atelocollagen-[CRO-Gem conjugate] to pancreatic cancer cell-lines.
FIG. 15 illustrates comparison results of breast cancer cell-line transmission efficacies between ERBB2 aptamer-siRNA conjugate and siRNA.
FIG. 16 illustrates *in vitro* efficacy examination results of ERBB2 aptamer-siRNA conjugate and siRNA to breast cancer cell-lines.
FIG. 17 illustrates *in vitro* efficacy examination results of atelocollagen-[ERBB2 aptamer-siRNA conjugate] complex to breast cancer cell-lines.
FIG. 18 illustrates *in vitro* efficacy examination results of AS1411-Dox adduct and CRO-Dox adduct to liver cancer cell-lines.
FIG. 19 illustrates *in vitro* efficacy examination results of atelocollagen-[AS1411-Dox adduct] complex to liver cancer cell-lines.
FIG. 20 illustrates measured results of tumor size over time after directly injecting 2.0 mg of AS1411-Gem conjugate and 0.5% atelocollagen complex to tumor in a mouse with pancreatic cancer via a syringe.
FIG. 21 illustrates measured results of tumor size over time after directly injecting atelocollagen alone to tumor in a mouse with pancreatic cancer via a syringe.
FIG. 22 is graphs illustrating a change in tumor size at different concentrations of atelocollgen-[AS1411-Gem conjugate] complex.
FIG. 23 is a photograph (FIG. 23A) and a graph (FIG. 23B) illustrating results of comparing sizes of excised tumors.
FIG. 24 is photographs showing atelocollagen-[AS1411-Gem conjugate] complex (patch type) and atelocollagen-[AS1411-Gem conjugate] complex (scaffold type) implanted around the tumor.
FIG. 25 is photographs (FIG. 25A) and graphs (FIG. 25B) illustrating comparison results of a change in tumor size over time after implanting the atelocollagen-[AS1411-Gem conjugate] complex (patch type, A), atelocollagen-[AS1411-Gem conjugate] complex (scaffold type, B), atelocollagen-[CRO-Gem conjugate] complex (patch type, C), and atelocollagen-[CRO-Gem conjugate] complex (scaffold type, D), respectively, around the tumors.
FIG. 26 is graphs illustrating comparison results of a change in tumor size over time after directly injecting atelocollagen-[ERBB2 aptamer-siRNA conjugate] complex into mice with breast cancer.
FIG. 27 is a photograph showing comparison of sizes of excited tumors.
FIG. 28 is graphs illustrating comparison results of a change in tumor size over time after implanting atelocollagen-[ERBB2 aptamer-siRNA conjugate] complex (patch type, A), atelocollagen-[ERBB2 aptamer-siRNA conjugate] complex (scaffold type, B), atelocollagen-[control ERBB2 aptamer-siRNA conjugate] complex (patch type, C), and atelocollagen-[control ERBB2 aptamer-siRNA conjugate] complex (scaffold type, D), respectively, around the tumors.
FIG. 29 is graphs illustrating comparison results of a change in tumor size over time after directly injecting atelocollagen-[AS1411-Dox adduct] complex (sol-gel type) and atelocollagen-[CRO-Dox adduct] complex (sol-gel type), respectively, into mice with liver cancer.
FIG. 30 is a photograph showing comparison of sizes of excited tumors.

### [Best mode]

A drug stabilizing complex of the present invention may include an atelocollagen-[aptamer-L-X conjugate] complex. Herein, X denotes any substance with therapeutic effects such as anticancer effects, anti-inflammatory effects, etc., for example, drugs, toxins, siRNA, peptides, protein drugs and nano-materials or the like, and may be used in a variety of applications without limitation thereof so long as the substance has therapeutic effects.

When being conjugated as aptamer-L-X (aptamer-drug conjugate), the aptamer has a targeting function and thus delivers the drug to a target site such as a cancer cell or a site of inflammation. If the above conjugate is combined with atelocollagen, the resultant product may be stabilized inside a body thus to exhibit further improved therapeutic effects.

Amounts of atelocollagen, aptamer and an anticancer active substance X may depend upon types of aptamers, the weight of a patient, a size of tumor to be treated, or a distance between tumor and the skin, etc., which can be appropriately adjusted.

The atelocollagen-[aptamer-L-X] complex (i.e., atelocollagen-[aptamer-drug conjugate] complex) of the present invention is not particularly limited to specific aptamers, but may be produced in the form of 'atelocollagen-[aptamer-L-X conjugate] complex (i.e., atelocollagen-[aptamer-drug conjugate] complex)' using different aptamers. In a case of producing in a form of a composite (that is, 'complex'), improvement in stability and anticancer effects of the aptamer may be expected. The complex of the present invention may be useful in treatment of diverse cancers including pancreatic cancer, breast cancer, liver cancer, etc. depending upon types of aptamers and anticancer active materials. Of course, the complex of the present invention may be applied to other therapeutic agents such as arthritis medicines.

The composition of the present invention may be used in a variety of formulations including, for example: sol-gel type; injection products which can be directly put into cancer or arthritic sites to enable solidification by a body temperature and to allow the drug to be slowly released from the same; transplant materials such as patch, implants, etc. which can be attached to a desired site, or the like. Therefore, the composition of the present invention may be useful to, in particular, decrease a tumor size before surgery and/or remove the remaining cancer cells after surgery without side effects or repulsion during radiation therapy.

### [Preferred Embodiments of Invention]

The present invention will be described in more detail by the following examples.

### Example 1. Preparation of high concentration atelocollagen dispersion for medical use

After adding 3 wt.% of a high concentration atelocollagen to NaOAc/HAc solution (1.2 g CH₃COONa, 27.5 ml CH₃COOH in 50 ml), the mixture was stirred and completely dissolved while maintaining pH 3.0. The solution was subjected to dia-filtration in PBS solution through tangential flow filtration (TFF), thereby preparing an atelocollagen dispersion for medical use in PBS solution.

### Aptamer-linker-drug conjugate synthesis

### Example 2. Synthesis of anti-nucleolin GRO aptamer-linker-gemcitabine conjugate [AS1411-Gem conjugate]

By reacting maleimidecarproyl-(Gly-Phe-Leu-Gly)-gemcitabine [MC-GFLG-Gemcitabine] with HS-C6-tttggtggtggtggttgtggtggtggtgg [HS-C6-T₃-AS1411], gemcitabine-(Gly-Leu-Phe-Gly)-Mal-S-C6-tttggtggtggtggttgtggtggtggtgg [Gemcitabine-(GLFG-MC-S-C6)-T₃-AS1411, AS1411-Gem conjugate] was synthesized. In other words, RSS-C6-tttggtggtggtggttgtggtggtggtgg [RSS-C6-T₃-AS141]) was subjected to reductive reaction in the presence of DTT for about 3 hours, and the remaining DTT was removed by Centricon and replaced with an SB17 buffer solution. After putting Mal-GPLG-Gemcitabine dissolved in a small amount of DMSO into the resultant product, the mixture was shaken overnight. Purification was performed through reverse-phase HPLC (Waters-Xbridge OST C18 10 X 50 mm, 65, TEAE/ACN buffer), thereby yielding Gemcitabine-(GLFG-MC-S-C6)-T₃-AS1411 [AS1411-Gem conjugate]. Through ESI-MS, a molecular weight of Gemcitabine-(GLFG-MC-S-C6-T₃-AS1411 [AS1411-Gem conjugate] was determined. C₃₃₄H₄₂₂F₂N₁₁₇O₁₉₈P₂₉S [Cal. MW = 10211.90 Obs. MW = 10211.87]

### Example 3. Synthesis of anti-nucleolin CRO aptamer-linker-gemcitabine conjugate [CRO-Gem conjugates]

By reacting maleimidecarproyl-(Gly-Phe-Leu-Gly)-gemcitabine [MC-GFLG-Gemcitabine] with HS-C6-tttcctcctcctccttctcctcctcctcc [HS-C6-T₃-CRO], gemcitabine-(Gly-Leu-Phe-Gly)-Mal-S-C6-tttcctcctcctccttctcctcctcctcc [Gemcitabine-(GLFG-MC-S-C6)-T₃-CRO, CRO-Gem conjugate] was synthesized. In other words, RSS-C6-tttcctcctcctccttctcctcctcctcc [RSS-C6-T₃-CRO] was subjected to reductive reaction in the presence of DTT for about 3 hours, and the remaining DTT was removed by Centricon and replaced with an SB17 buffer solution. After putting Mal-GPLG-Gemcitabine dissolved in a small amount of DMSO into the resultant product, the mixture was shaken overnight. Purification was performed through reverse-phase HPLC (Waters-Xbridge OST C18 10 X 50 mm, 65, TEAE/ACN buffer), thereby yielding gemcitabine-(GLFG-MC-S-C6)-T₃-CRO [CRO-Gem conjugate]. Through ESI-MS, a molecular weight of gemcitabine-(GLFG-MC-S-C6)-T₃-CRO [CRO-Gem conjugate] was determined. C₃₁₇H₄₂₂F₂N₈₃O₁₉₈P₂₉S [Cal. MW = 9531.49 Obs. MW = 9532.09].

### Example 4. Synthesis of anti-nucleolin GRO aptamer-doxorubicin adduct [AS1411-Dox adduct]

By reaction AS1411 with doxorubicin-HCl (adramycin-HCl), AS1411-Dox adduct was synthesized. In other words, 20 µM of GRO, 150 µM of doxorubicin-HCl and 0.37% formaldehyde were put into 0.5 ml of buffer solution (20 mM sodium phosphate, 150 mM NaCl, 0.5 mM EDTA; pH 7.0) and subjected to reaction while shaking overnight at 10°C. After the reaction, separation/purification were performed through reverse-phase HPLC (Waters-Xbridge OST C18 10 X 50 mm, 0.1 M TEAE/ACN buffer, 260 nm, 490 nm). After the separation/purification, the resultant product was concentrated and desalted by Centricon (Millipore, MW 3000 cut).

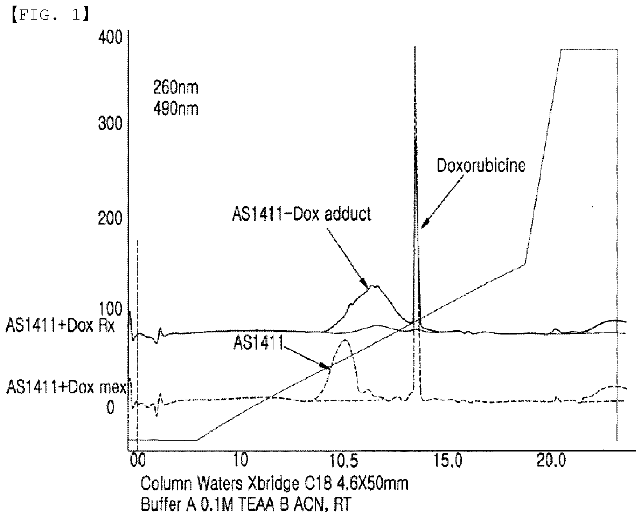

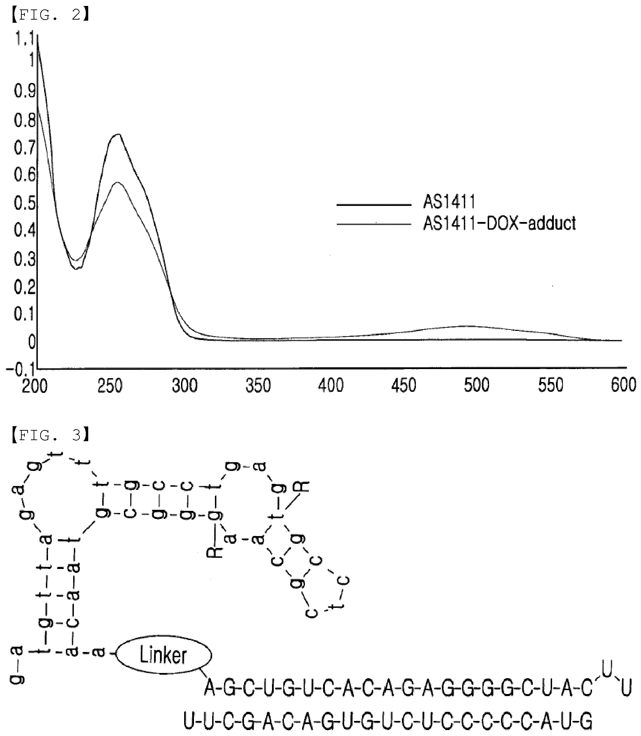

AS1411-Dox adduct was identified by HPLC retention time, UV-Vis spectra and ESI-MS (FIG. 1 and FIG. 2).

### Example 5. Synthesis of CRO aptamer-doxorubicin adduct [CRO-Dox adduct]

By reacting a control aptamer with doxorubicin-HCl (Adramycin-HCl), a control-Dox adduct was synthesized. In other words, 20 µM of control aptamer, 150 µM of doxorubicin-HCl and 0.37% formaldehyde were put into 0.5 ml buffer (20 mM sodium phosphate, 150 mM NaCl, 0.5 mM EDTA; pH 7.0) and subjected to reaction while shaking overnight at 10°C. After the reaction, separation/purification were performed through reverse-phase HPLC (Waters-Xbridge OST C18 10 X 50 mm, 0.1 M TEAE/ACN buffer, 260 nm, 490 nm). After the separation/purification, the resultant product was concentrated and desalted by Centricon (Millipore, MW 3000 cut) .

CRO-Dox adduct was identified through HPLC retention time, UV-Vis spectra and ESI-MS.

### Example 6. Synthesis of ERBB2 aptamer-siRNA conjugate

From ERBB2 aptamer [a6g66agag666gcc6gag6gcc6cgcaagggcg6aacaa, 6 = NapdU [5-(N-naphthylcarboxyamide)-2'-deoxyuridine]] described in Patent Application Nos. KR10-1279584 and US9309515B2 filed in the name of the present inventors, ERBB2 aptamer-siRNA conjugate was synthesized. The siRNA targeting Bcl-2 in a cell was synthesized so as to have a maleimide group at the terminal part thereof. Further, the terminal of the ERBB2 aptamer was synthesized by modifying it into a thiol group. The thiolated aptamer and maleimide-siRNA were reacted with 8 mM sulfo-SMPB, and the ERBB2 aptamer was activated by a reaction with 10 mM TCEP dissolved in PBS at 37°C for 1 hour. The above two materials were prepared after purification through Centri-Sep column. These materials were fully mixed, followed by reaction at 37°C for 2 hours. Finally, these two materials, which have completed conjugation, were subjected to PAGE purification and quantification by a UV spectrophotometer (FIG. 3)

### Preparation of atelocollagen-[aptamer-drug conjugate] complex

### Example 7. Preparation of ateolocollagen-[aptamer-drug conjugate] complex composition (sol-gel type)

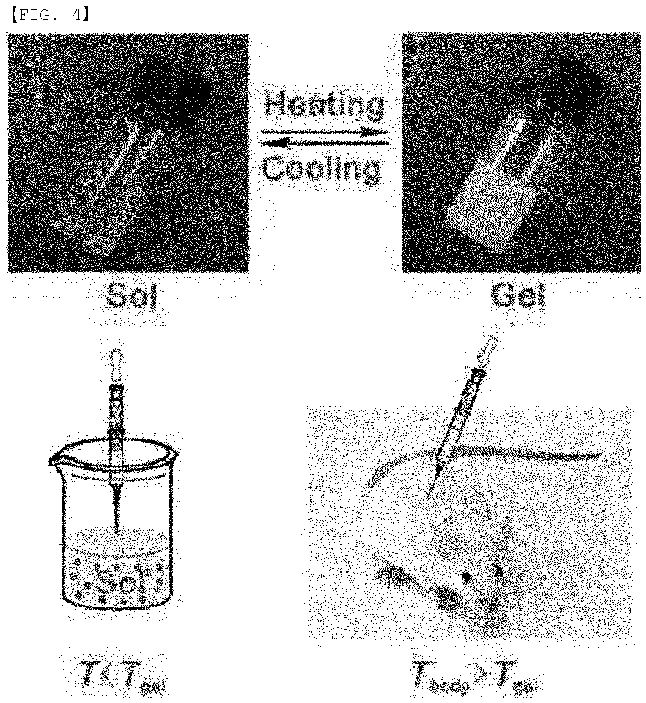

The aptamer-drug conjugate prepared through synthesis was put into PBS, and allowed to be completely dissolved using a mixer at room temperature. The reagent prepared as described above was mixed with a high concentration atelocollagen dispersion at an appropriate concentration of 0.5 to 3.0%, and then processed by a rotator cuff device at room temperature for 30 minutes to prepare a mixture solution. The atelocollagen-[aptamer-drug conjucate] complex (sol-gel type) is in a liquid state (sol) at a low temperature. On the other hand, this complex is solidified to form a gel at 37°C when being directly injected into tumors *in vivo,* so that the atelocollagen surrounding the aptamer-drug conjugate (sol-gel type) is gradually molten and the drug may be slowly released to serve as an effective tumor therapeutic agent (FIG. 4).
1) Pancreatic cancer: Atelocollagen-[AS1411-Gem conjugate] complex (sol-gel type)
2) Breast cancer: Atelocollagen-[ERBB2 aptamer-siRNA conjugate] complex (sol-gel type)
3) Liver cancer: Atelocollagen-[AS1411-Dox adduct] complex (sol-gel type)

### Example 8. Preparation of atelocollagen-[aptamer-drug conjugate] complex composition (patch type)

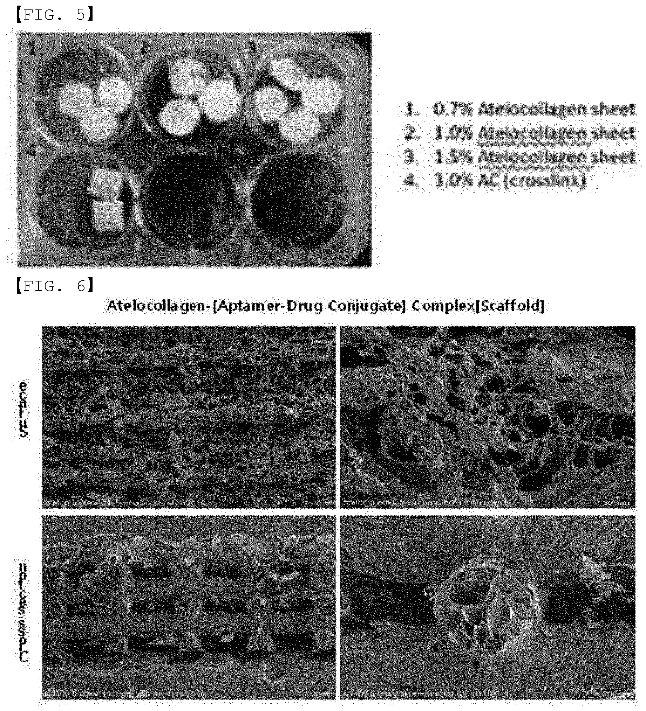

A mixture solution was prepared using the high concentration collagen dispersion prepared above and the synthesized aptamer-drug conjugate in the same manner as the formation of a sol-gel type product. After putting the prepared mixture solution into a 48-well cell culture plate and diffusing the same to form a uniform film with a thickness of 0.5 mM, the film was frozen at minus (-) 80°C. The frozen sample was lyophilized in a lyophilizer maintaining at -70°C for 30 hours, thereby yielding a porous membrane (FIG. 5).

### [Example 9. Preparation of atelocollagen-[aptamer-drug conjugate] complex composition (scaffold type).

Using an aptamer-drug conjugate synthesized with an atelocollagen dispersion as a bio-compatible and biodegradable natural polymer, an atelocollagen-[aptamer-drug conjugate] complex having a three-dimensional (3D) structure (scaffold type) was prepared. The aptamer-drug conjugate generated by degradation of atelocollagen was combined with cancer cells so that the conjugate could selectively act on the cancer cells. In the preparation, a solid-free form fabrication process was adopted in 3D plotter. Further, the preparation method included: introducing the atelocollagen-[aptamer-drug conjugate] complex mixture solution to a barrel equipped with a precise nozzle (diameter: 300 µm); and fixing the barrel to a plate at 37°C using a 3D plotter at a discharge pressure of 300 ± 50 kPa with a floating rate of 5 mm/s, thereby fabricating 3 layers of 3D atelocollagen-[aptamer-drug conjugate] complex (scaffold type).

In addition, a cell carrier in a grid structure was formed to increase pore efficiency, and the atelocollagen was prepared in different concentrations (0.5, 1.5, and 3.0 wt.%), thus to produce 3D atelocollagen-[aptamer-drug conjugate] complex (scaffold type) that can control a mechanical strength or degradation rate of the cell carrier. For cross-linkage between the atelocollagen-[aptamer-drug conjugate] complex (scaffold type) and the cell carrier, genipin was used. More particularly, the above two substances were cross-linked in 1% genipin solution under a condition of 20°C for 24 hours (FIG. 6).

### In vivo stability verification

### Example 10. In vivo stability experiment of atelocollagen-[aptamer-drug conjugate] complex composition (sol-gel type).

**[TABLE 1]**

| Number | Treat | Treat concentration |
|---|---|---|
| 1 | Negative control | 2.0% Ateloollagen |
| 2 | Positive control | 10 µM Cy3-AS1411-Gem Conjugate |
| 3 | Sample (1) | 10 µM Cy3-AS1411-Gem Conjugate & 2.0% Atelocollagen |
| 4 | Sample (2) | 10 µM Cy3-AS1411-Gem Conjugate & 0.3% Atelocollagen |

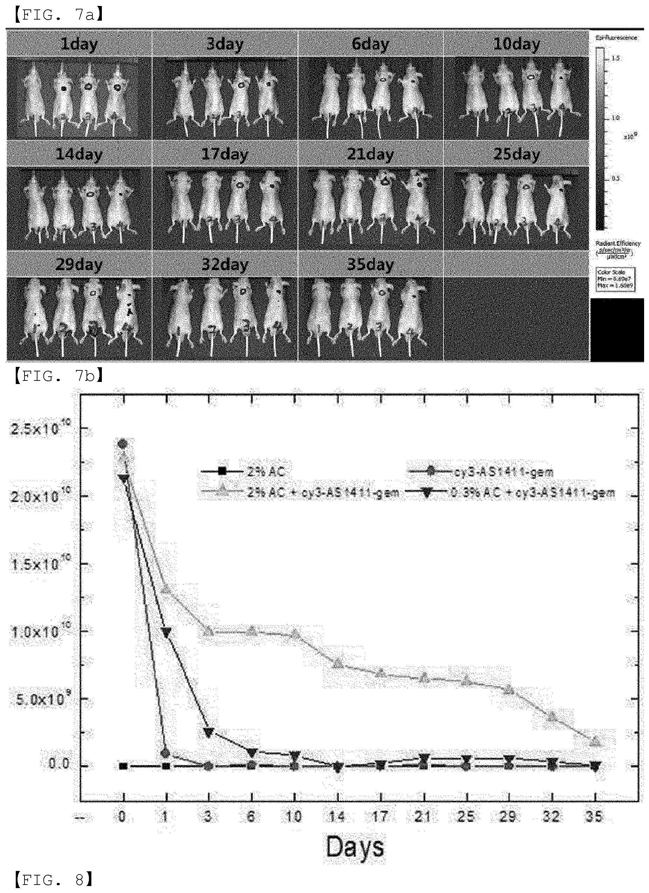

Cy3-labelled AS1411-Gem conjugate [Cy3-AS1411-Gem conjugate] and atelocollagen-[Cy3-AS1411-Gem conjugate] complex (sol-gel type) were prepared at specific concentrations as shown in the above table. Each of the pretreated Cy3-AS1411-Gem conjugate and atelocollagen-[Cy3-AS1411-Gem conjugate] complex (sol-gel type) was injected into the subcutaneous layer of each nude mouse. Thereafter, according to a method for measuring a brightness level of fluorescence by date, it was observed how much Cy3-AS1411-Gem conjugate is maintained in a stable state. For this purpose, IVIS, which is a fluorescence measurement device, was used to measure the fluorescent level at intervals of 3 to 4 days. Through analysis of the obtained results, it could be seen that, when Cy3-AS1411-Gem conjugate alone was injected into an animal, the conjugate was substantially spread from a body site into which the above conjugate was introduced to the whole body of the animal, and the fluorescence disappeared within 1 day. On the other hand, when the atelocollagen-[Cy3-AS1411-Gem conjugate] complex (so-gel type) and the atelocollagen were injected together, it was observed that the conjugate at a low concentration was slowly spread over several days whereas the Cy3-AS1411-Gem conjugate at a high concentration still remained in a considerable amount on the injection site for maximum 35 days. Accordingly, in a case of a complex of the Cy3-AS1411-Gem conjugate and the atelocollagen, it was demonstrated that the complex could quite stably remain *in vivo,* compared to administration of the conjugate alone (FIG. 7A and FIG. 7B).

### Example 11. In vivo stability experiment of atelocollagen-[aptamer-drug conjugate] complex composition (patch type)

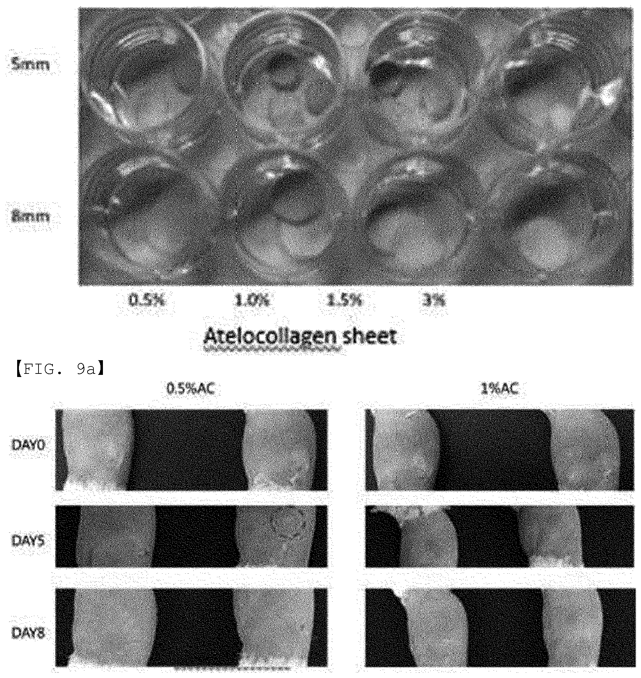

Cy3-labelled AS1411-Gem conjugate [Cy3-AS1411-Gem conjugate] and atelocollagen-[Cy3-AS1411-Gem conjugate] were lyophilized to form sheets. The sheets were fabricated in a size of 5 mm and 8 mm using each of atelocollagens at concentrations of 0.5%, 1.5% and 3.0% (FIG. 8).

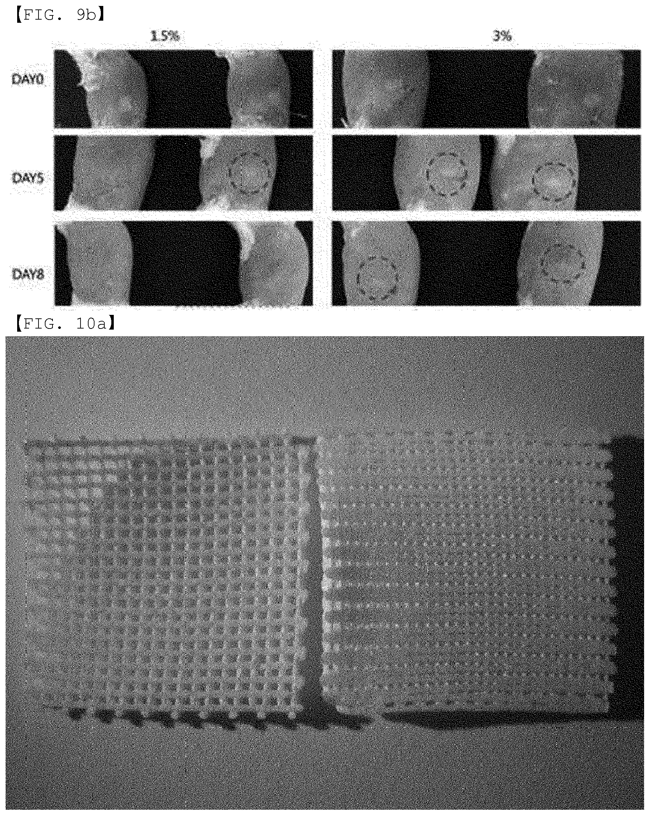

After transplanting the prepared sheets on the outer skin layer of each mouse, degradation rates were visually measured. The sheets at 0.5% and 1% concentrations were observed to be molten and absorbed within 5 days. Further, the sheet at 1.5% concentration partially remained after 5 days and was completely molten on day 8. Further, the sheet at 3% concentration was observed to remain without being completely molten even after 8 days (FIG. 9).

### Example 12. In vivo experiment of atelocollagen-[aptamer-drug conjugate] complex composition (scaffold type)

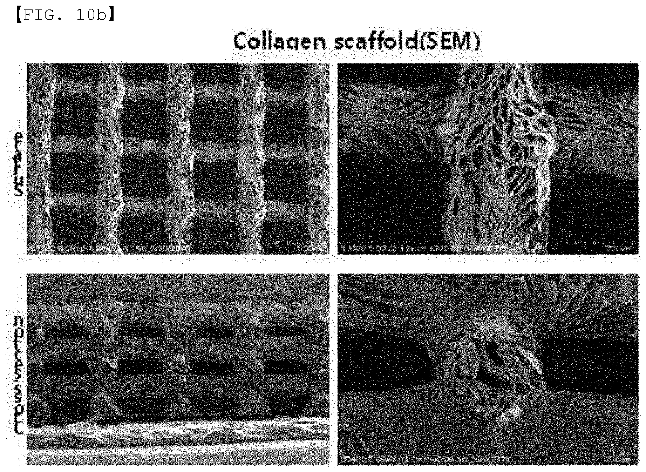

Cell carriers were fabricated in a 3D grid structure using atelocollagen by means of a 3D plotter. More particularly, the cell carriers were fabricated in sizes of 5 × 5 × 2 mm³ and 10 × 10 x 2 mm³ using the atelocollagen at concentrations of 0.5%, 1.5% and 3.0%. Further, in order to control a degradation rate *in vivo,* the cell carriers were cross-linked in a 1-ethyl-(3-3-dimethylaminopropyl) carbodiimide hydrochloride solution (EDC, Sigma Chemical Co., St. Louis, MO, USA) for 24 hours. Otherwise, the cell carriers were cross-linked using genipin as a bio-compatible material to form a scaffold. The scaffold obtained as described above was washed with distilled water using an ultrasonic mill, followed by lyophilization at -50°C (FIG. 10A and FIG. 10B).

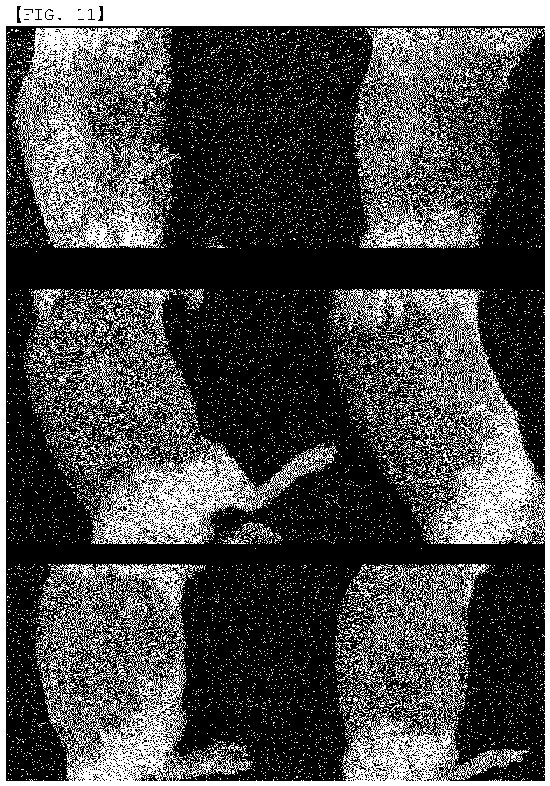

The cross-linked scaffold was transplanted on the outer skin layer of a mouse in the same manner as the method performed above, and was observed over time. It was confirmed that no substantial degradation was observed even at a time of 8 days passed after transplantation. Therefore, it was demonstrated that the degradation rate was prominently reduced due to the cross-linkage (FIG. 11).

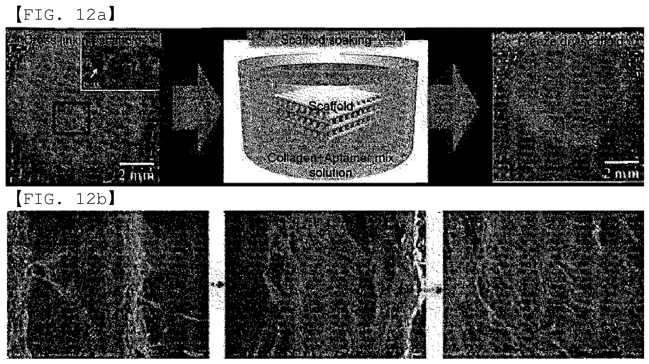

Cy3-AS1411-Gem conjugate was carried on the scaffold to be observed by an electron microscope after (FIG. 12).

### In vitro efficacy validation

### Example 13. In vitro efficacy of aptamer-drug conjugate to pancreatic cancer

WST-1 assay for pancreatic cancer cell lines, i.e., BxPC3, Miapaca-2, Panc-1 and capan-1 was performed. Cell inhibition efficacy validation of AS1411-Gem conjugate to the pancreatic cancer cell lines was performed *in vitro* through WST-1 assay. First, a control aptamer CRO in a naked form without being combined with an anticancer agent, AS1411 having a cancer cell-targeting function, as well as CRO-Gem conjugate and AS1411-Gem conjugate which are combined with an anticancer agent effective to pancreatic cancer, i.e., gemcitabine, respectively, were prepared (Table 2).

**[TABLE 2]**

| Name | SEQUENCE | MW |
|---|---|---|
| AS1411 | TTTGGTGGTGGTGGTTGTGGTGGTGGTGG | 9185.01 |
| CRO | TTTCCTCCTCCTCCTTCTCCTCCTCCTCC | 8504.50 |
| AS1411-Gem conjugate | (Gem) ₙ -L-GGTGGTGGTGGTTGTGGTGGTGGTGG | 8576.61 |
| CRO-Gem conjugate | (Gem) ₙ -L-CCTCCTCCTCCTTCTCCTCCTCCTCC | 7896.10 |

### Gem = gemcitabine, n = 1 to 10

In comparison of cell viability and proliferation of the cells through WST-1 assay, it was confirmed that AS1411 aptamer has much better efficacy than CRO as the control aptamer, and further showed more excellent efficacy than the case of treatment using the original anticancer agent, that is, gemcitabine alone.

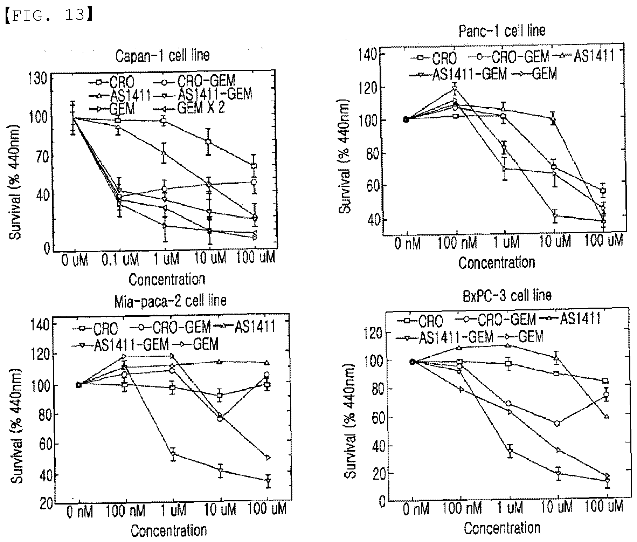

Experimental procedure: Each of the pancreatic cancer cell lines, that is, B x PC3, Miapaca-2, Panc-1 and capan-1 (ATCC, RPMI + 10% FBS), was seeded on a 96-well plate in a cell number of 1 to 2 x 10⁴ cells/well, which was defined by a cell test method to determine an appropriate cell concentration, followed by growing for 1 day. The aptamer and aptamer-drug conjugate were dissolved in PBS, respectively, and were immediately treated in each well at concentrations from 0.1µM up to 100 µM. The treated cells were incubated in a 5% CO₂ incubator for 72 hours, treated with 10µl of a reagent solution for WST-1 assay (WST-1, Roche), respectively, followed by measuring the absorbance at 440 nm using an ELISA reader (FIG. 13).

### Example 14. In vitro efficacy of atelocollagen-[aptamer-drug conjugate] complex to pancreatic cancer

WST-1 assay for the pancreatic cancer cell lines, i.e., Miapaca-2 and MCF-7 was performed. Cell inhibition efficacy validation of the atelocollagen-[AS1411-Gem conjugate] complex to the pancreatic cancer cell lines was performed *in vitro* through WST-1 assay. First, a control aptamer CRO in a naked form without being combined with an anticancer agent, AS1411 having a cancer cell-targeting function, as well as atelocollagen-[AS1411-Gem conjugate] complex and atelocollagen-[CRO-Gem conjugate] complex which are combined with an anticancer agent effective to pancreatic cancer, i.e., gemcitabine, respectively, were prepared.

In comparison of cell viability and proliferation of the cells through WST-1 assay, it was confirmed that the atelocollagen-[AS1411-Gem conjugate] complex has much better efficacy than the atelocollagen-[CRO-Gem conjugate] complex, and further showed more excellent efficacy than the case of treatment using the original anticancer agent, that is, gemcitabine alone.

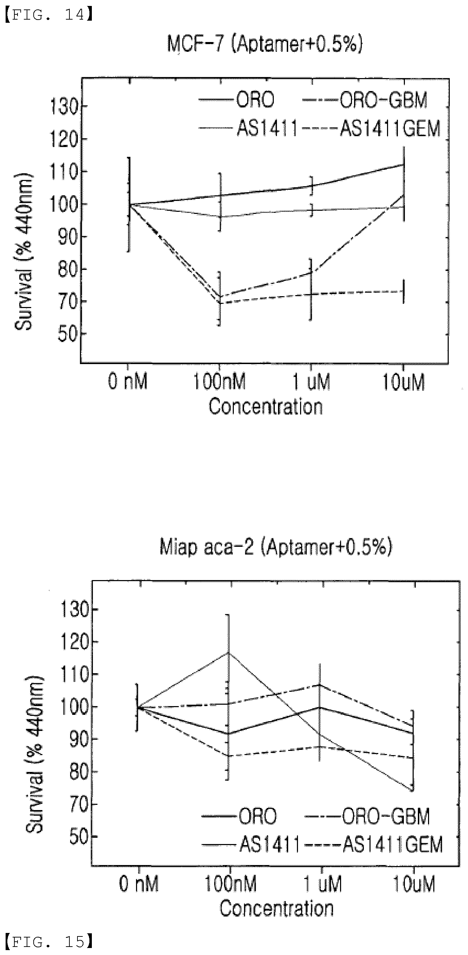

Experimental procedure: Each of the pancreatic cancer cell lines, that is, Miapaca-2 and MCF-7 (ATCC, RPMI + 10% FBS), was seeded on a 96-well plate in a cell number of 1 to 2 x 10⁴ cells/well, which was defined by a cell test method to determine an appropriate cell concentration, followed by growing for 1 day. The aptamer and atelocollagen-[Aptamer-drug conjugate] complex were dissolved in PBS, respectively, and were immediately treated in each well at concentrations from 0.1µM up to 100 µM. The treated cells were incubated in a 5% CO₂ incubator for 72 hours, treated with 10µl of a reagent solution for WST-1 assay (WST-1 Roche), respectively, followed by measuring the absorbance at 440 nm using an ELISA reader (FIG. 14).

### Example 15. In vitro efficacy of ERBB2 aptamer-drug conjugate to breast cancer

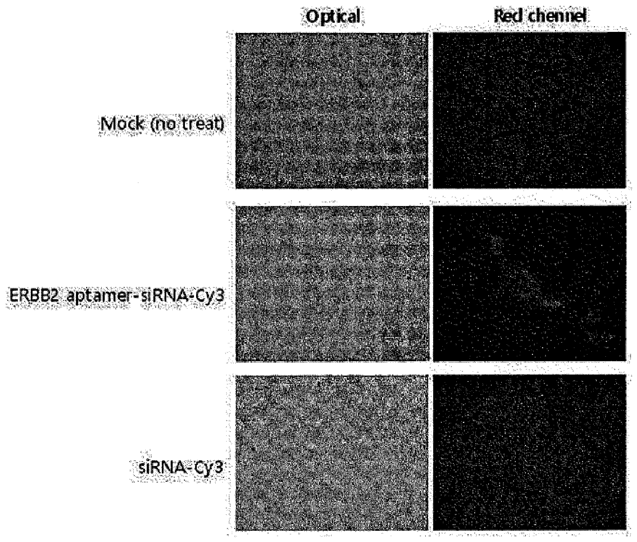

In order to identify targeting to a breast cancer target cell, ERBB2 aptamer-linker-siRNA-Cy3, which was modified with Cy3 at siRNA, and siRNA-Cy3 as a control group were prepared. ERBB2 over-expressed MCF7 cell lines were treated with these two synthesized materials, respectively, followed by observation through a fluorescence microscope. In a case of the siRNA control groups including untreated one and the other only combined with Cy3, the cells did not show fluorescence. On the other hand, the cells exhibited fluorescent in the test group combined with ERBB2 aptamer only. By analyzing such results, it could be found that siRNA might be accurately delivered to target cells through targeting by aptamer (FIG. 15)

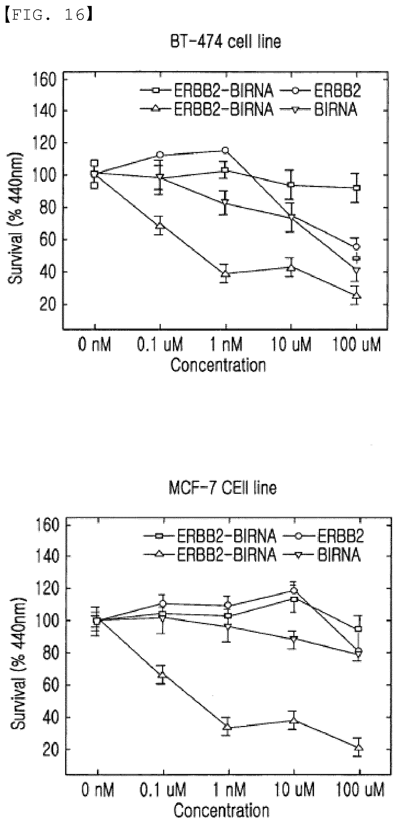

In addition, WST-1 assay for the breast cancer cell lines, i.e., BT-474 and MCF-7, was performed. Cell inhibition efficacy validation of ERBB2 aptamer to the breast cancer cell lines was performed *in vitro* through WST-1 assay. After treating the breast cancer cell lines with a control ERBB2 aptamer without activity, as well as ERBB2 aptamer-siRNA conjugate wherein an effective anticancer agent having excellent anticancer efficacy, that is, siRNA is bound to ERBB2 target aptamer having a cancer cell targeting function, respectively, WST-1 assay was performed. In comparison of cell viability and proliferation of the cells, it was demonstrated that the ERBB2 aptamer-siRNA conjugate has much better efficacy than the ERBB2 aptamer, thereby verifying excellent efficacy in the breast cancer cell line (FIG. 16).

### Example 16. In vitro efficacy of atelocollagen-[aptamer-drug conjugate] complex to breast cancer

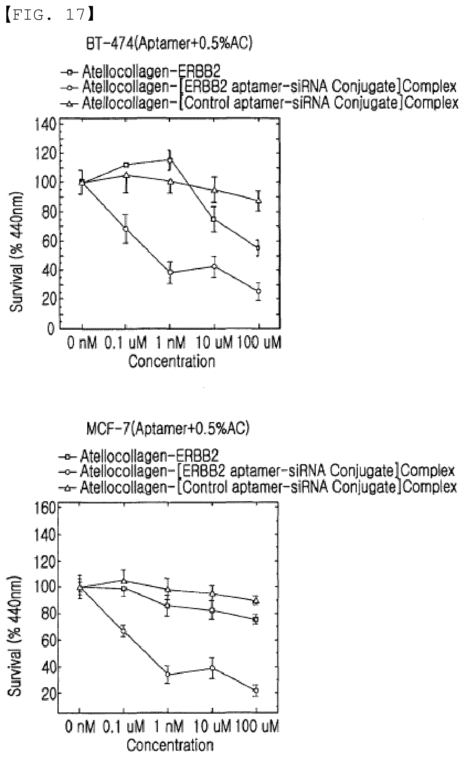

Cell inhibition efficacy validation of atelcollagen-[ERBB2 aptamer-siRNA conjugate] complex to breast cancer cell lines, i.e., BT-474 and MCF-7, was performed *in vitro* through WST-1 assay. After treating the breast cancer cell lines with naked ERBB2, atelocollagen-[control aptamer-siRNA conjugate] complex without activity, as well as atelocollagen-[ERBB2 aptamer-siRNA conjugate] complex wherein an effective anticancer agent having excellent anticancer efficacy, that is, siRNA is bound to ERBB2 target aptamer having a cancer cell targeting function, respectively, WST-1 assay was performed. In comparison of cell viability and proliferation of the cells, it was confirmed that the ERBB2 aptamer-siRNA conjugate has much better efficacy than the control group, that is, the atelocollagen-[control aptamer-siRNA conjugate] complex aptamer, thereby verifying excellent efficacy in the breast cancer cell line (FIG. 17).

### Example 17. In vitro efficacy of AS1411-Dox adduct to liver cancer

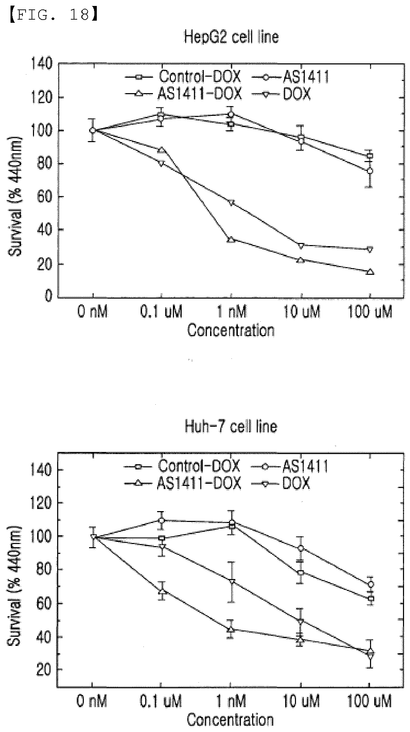

In the same manner as executed above, cell inhibition efficacy validation of AS1411-Dox adduct as an aptamer-drug conjugate as well as a control-Dox adduct as a control aptamer to breast cancer cell lines, i.e., HepG2 and Huh-7, was performed *in vitro* through WST-1 assay. From the result, it was confirmed that the AS1411-Dox adduct has much better efficacy than the control-Dox adduct, thereby verifying active performance of the composition (FIG. 18).

### Example 18. In vitro efficacy of atelocollagen-[aptamer-drug conjugate] complex to liver cancer

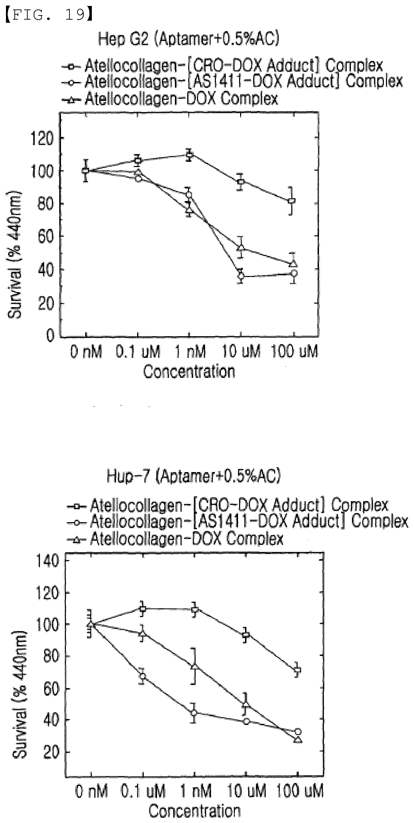

In the same manner as described in Example 17, the liver cell lines were prepared, and the prepared breast cancer cell lines were treated with atelocollagen-[AS1411-Dox adduct] complex having anticancer effects, atelocollagen-[CRO-Dox-adduct] complex without a targeting function and atelocollagen-Dox, respectively, followed by performing the efficacy validation through WST-1 assay. As predicted, the results showed that the atelocollagen-[AS1411-Dox adduct] complex having a targeting function has prominent efficacy, as compared to the control group, that is, the atelocollagen-[CAO-Dox-adduct] complex. Further, in a case of administration of Dox alone as well as collagen, cell inhibition due to non-selective cell permeation was observed (FIG. 19).

### In vivo efficacy validation

### Example 19. Efficacy validation of atelocollagen-[AS1411-Gem conjugate] complex (sol-gel type) in pancreatic cancer animal model

Preparation of animal model: 6 week-old Balb/c nude female mice were prepared as *in vivo* tumor models. An average weight of the mice used in the experiments was 20 g. Human-derived pancreatic cancer cell lines, that is, capan-1 cells were purchased from ATCC Co., and proliferated. By subcutaneous injection of the proliferated capan-1 cells into the mice in an amount of as many as 5 X 10⁷ cells to form a tumor. By observing every day in 2 to 3 weeks after the injection, the growth of tumor was visually checked and a size of the tumor was measured using a caliper.

Efficacy Validation of the complex: After the tumor has grown to an appropriate size, each of the atelocollagen-[AS1411-Gem conjugate] complexes (sol-gel type) as shown in Table 3 below, was directly injected to a site of the grown tumor in each mouse through a syringe (Table 3).

**[TABLE 3]**

| **Composition of atelocollagen-[AS1411-Gem Conjugate] Complex (Sol-Gel type)** | **Treat number** | **Number of surviving mice** |
|---|---|---|
| AS1411-Gem Conjugate 2.0 mg | 4 | 4 |
| AS1411-Gem Conjugate 1.5 mg | 4 | 4 |
| AS1411-Gem Conjugate 1.0 mg | 4 | 4 |
| AS1411-Gem Conjugate 0.5 mg | 4 | 4 |

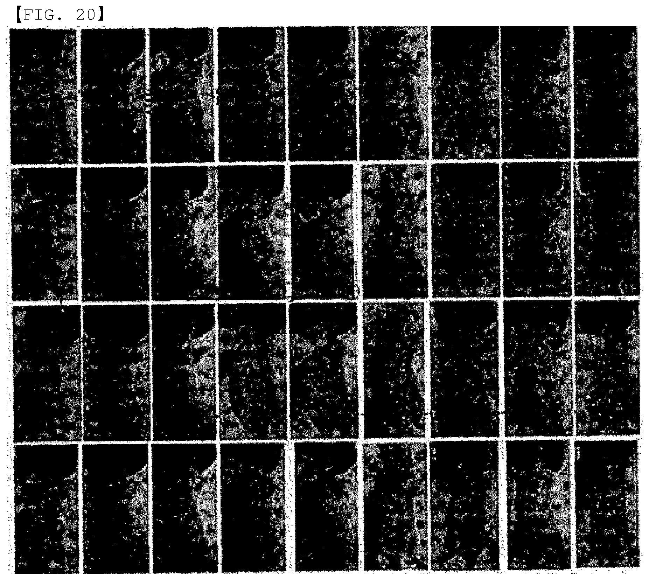

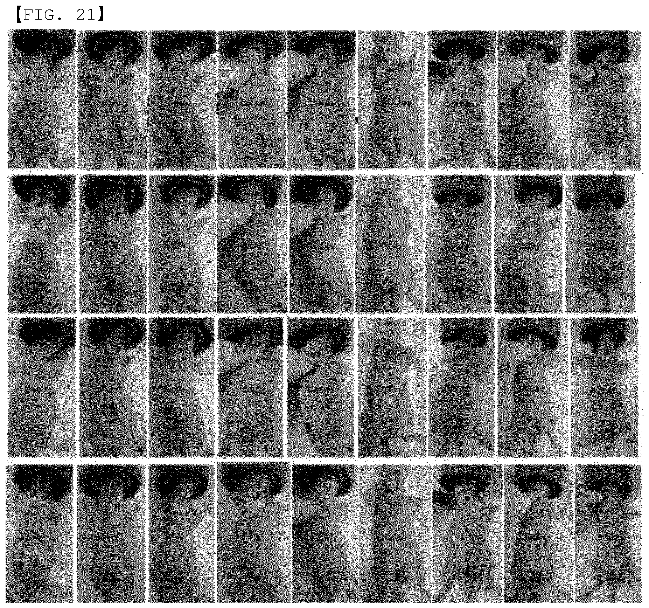

Further, in order to determine anticancer effects of the atelocollagen-[AS1411-Gem conjugate] complexes (sol-gel type), a change in the tumor size and a change in the weight of the implant were observed over a predetermined period of time (FIG. 20 and FIG. 21).

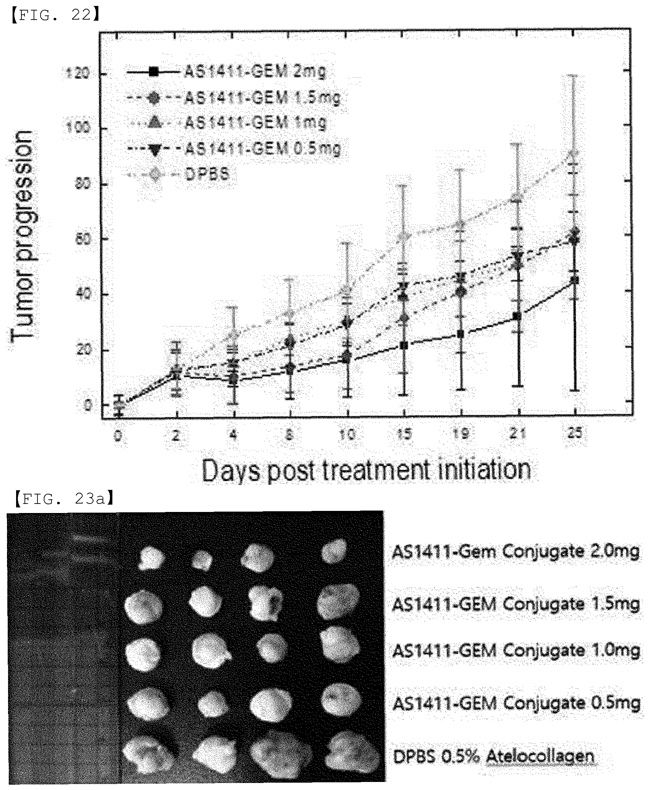

As compared to a control group, that is, a case of injecting 0.5% atelocollagen alone, the above experimental group exhibited prominent cancer-inhibitory efficacy, and it was observed that anticancer effects was increased with higher concentration (FIG. 22).

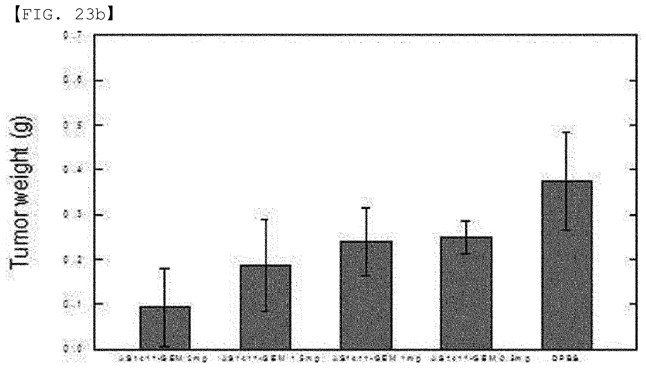

At a time of 30 days passed after the transplantation of the complex, the tumors were excited from each group. All excited tumors were subjected to measurement of weight and photographing, followed by comparing the actual tumor sizes to one another (FIG. 23).

### Example 20. Efficacy validation of atelocollagen-[AS1411-Gem conjugate] complex (patch type) and atelocollagen-[AS1411-Gem conjugate] complex (scaffold type) in pancreatic cancer animal model

**[TABLE 4]**

| **Treat concentration** | **Treat number** | **Number of surviving mice** |
|---|---|---|
| Atelocollagen-[AS1411-Gem Conjugate] Complex (patch type) | 3 | 3 |
| Atelocollagen-[AS1411-Gem Conjugate] Complex (scaffold type) | 3 | 3 |
| Atelocollagen-[CRO-Gem Conjugate] Complex (patch type) | 3 | 3 |
| Atelocollagen-[CRO-Gem Conjugate] Complex (scaffold type) | 3 | 3 |
| DPBS 0.5% Atelocollagen | 3 | 3 |

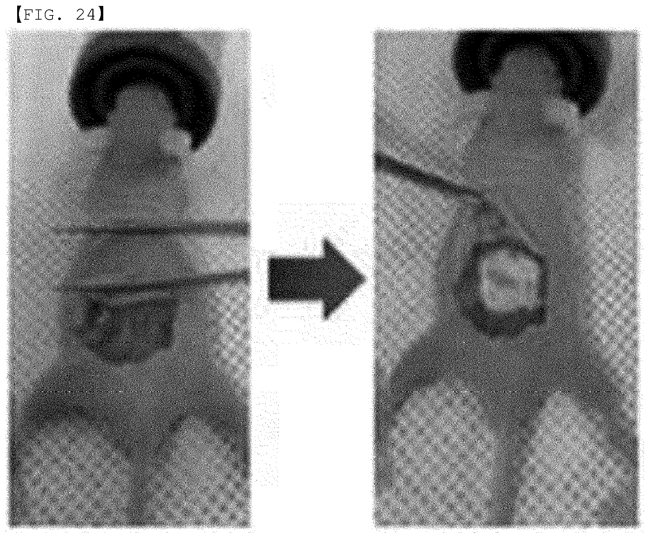

Tumor animal models were prepared in the same manner as described in Example 19, and then, the atelocollagen-[AS1411-Gem conjugate] complex (patch type) and atelocollagen-[AS1411-Gem conjugate] complex (scaffold type) prepared by the same method as described in Examples 8 and 9 were transplanted around the tumors in the tumor animal models, respectively (FIG. 24).

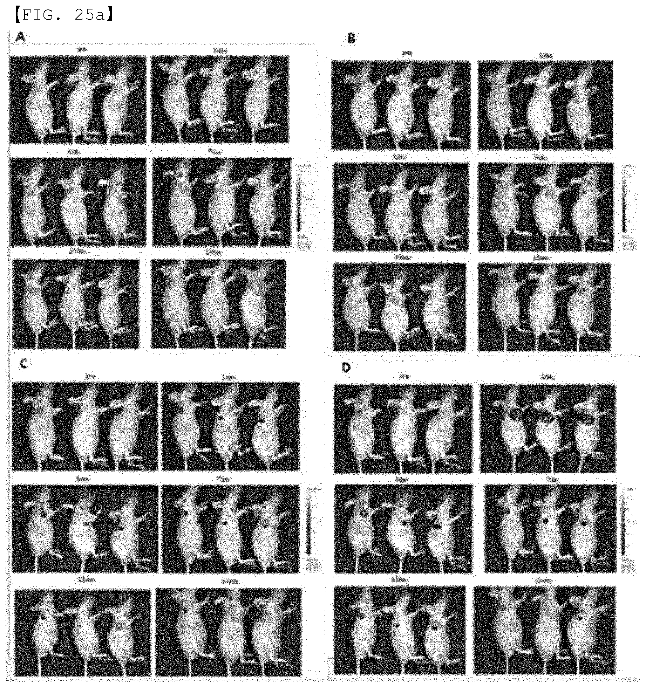

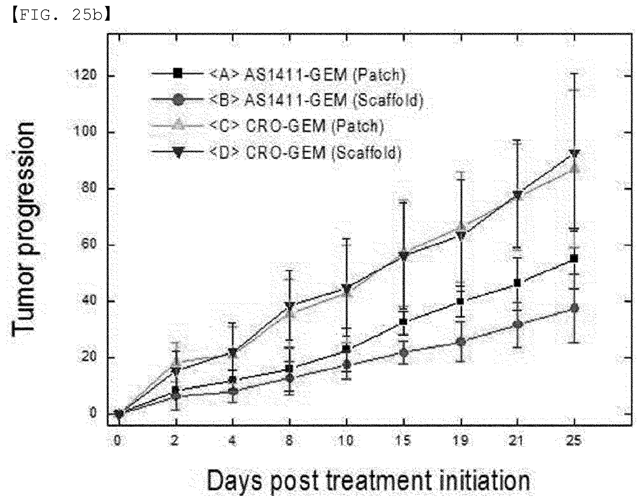

In order to determine anticancer effects of the transplanted atelocollagen-[AS1411-Gem conjugate] complex (patch type) and atelocollagen-[AS1411-Gem conjugate] complex (scaffold type), a change in the tumor size and a change in the weight of the implant were observed over a predetermined period of time. From the results, it was confirmed that the atelocollagen-[AS1411-Gem conjugate] complex (scaffold type) has much better anticancer efficacy than the atelocollagen-[AS1411-Gem conjugate] complex (patch type) (FIG. 25).

### Example 21. Efficacy validation of atelocollagen-[ERBB2 aptamer-siRNA conjugate] complex (sol-gel type) in breast cancer animal model

Preparation of animal model: 6 week-old Balb/c nude female mice were prepared as *in vivo* tumor models. An average weight of the mice used in the experiments was 20 g. A human-derived breast cancer cell lines, that is, BT-474 cells were purchased from ATCC Co., and proliferated. By subcutaneous injection of the proliferated BT-474 cells into the mice in an amount of as many as 1 X 10⁷ cells to form a tumor. By observing every day in 2 to 3 weeks after the injection, the growth of tumor was visually checked and a size of the tumor was measured using a caliper.

**[TABLE 5]**

| **Treat concentration** | **Treat number** | **Number of survival** |
|---|---|---|
| Atelocollagen-[ERBB2 aptamer-siRNA Conjugate] Complex (Sol-Gel type) ERBB2 aptamer-siRNA Conjugate 2 mg & 0.5% Atelocollagen | 3 | 3 |
| DPBS 0.5% Atelocollagen | 3 | 3 |

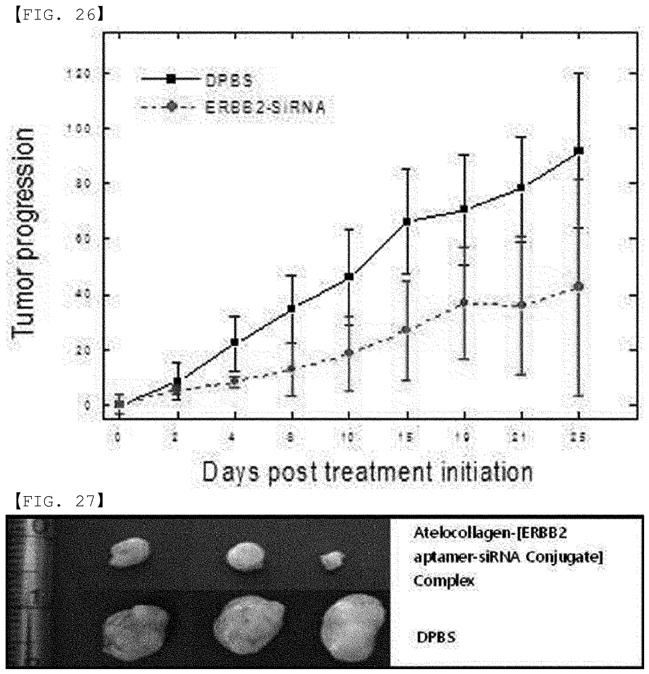

Efficacy validation of atelocollagen-[ERBB2 aptamer-siRNA conjugate] complex (sol-gel type): After the tumor has grown to an appropriate size, the atelocollagen-[ERBB2 aptamer-siRNA conjugate] complex (sol-gel type) as shown in Table 5 above, was directly injected to a site of the grown tumor in each mouse through a syringe. Further, in order to determine anticancer effects of the atelocollagen-[ERBB2 aptamer-siRNA conjugate] complex (sol-gel type), a change in the tumor size and a change in the weight of the implant were observed over a predetermined period of time. Prominent cancer-inhibitory efficacy was observed, as compared to the control group, that is, a case of injecting 0.5% atelocollagen alone (FIG. 26).

At a time of 30 days passed after the injection of atelocollagen-[ERBB2 aptamer-siRNA conjugate] complex (sol-gel type), the tumors were excited from each group. All excited tumors were subjected to photographing, followed by comparing the actual tumor sizes to one another (FIG. 27).

### Example 22. Efficacy validation of atelocollagen-[ERBB2 aptamer-siRNA conjugate] complex (patch type) and atelocollagen-[ERBB2 aptamer-siRNA conjugate] complex (scaffold type) in breast cancer animal model

Tumor animal models were prepared in the same manner as described in Example 21, and then, the atelocollagen-[ERBB2 aptamer-siRNA conjugate] complex (patch type) and atelocollagen-[ERBB2 aptamer-siRNA conjugate] complex (scaffold type) prepared by the same method as described in Examples 7 and 8 were transplanted around the tumors in the tumor animal models, respectively.

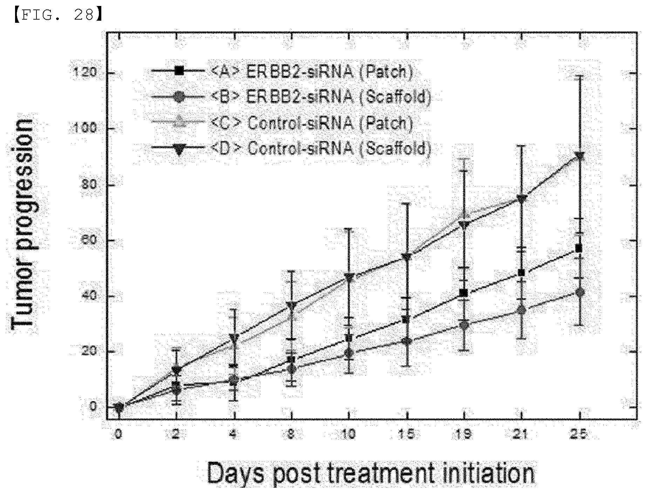

In order to determine anticancer effects of the transplanted atelocollagen-[ERBB2 aptamer-siRNA conjugate] complex (patch type) and atelocollagen-[ERBB2 aptamer-siRNA conjugate] complex (scaffold type), a change in the tumor size and a change in the weight of the implant were observed over a predetermined period of time. From the results, it was confirmed that the atelocollagen-[ERBB2 aptamer-siRNA conjugate] complex (scaffold type) has much better anticancer efficacy than the atelocollagen-[ERBB2-Gem conjugate] complex (patch type) (FIG. 28).

### Example 23. Efficacy validation of atelocollagen-[AS1411-Dox adduct] complex (sol-gel type) in liver cancer animal model

Preparation of animal model: 6 week-old Balb/c nude female mice were prepared as *in vivo* tumor models. An average weight of the mice used in the experiments was 20 g. A human-derived liver cancer cell lines, that is, Huh-7 cells were purchased from ATCC Co., and proliferated. By subcutaneous injection of the proliferated Huh-7 cells into the mice in an amount of as many as 5 X 10⁶ cells to form a tumor. By observing every day in 2 to 3 weeks after the injection, the growth of tumor was visually checked and a size of the tumor was measured using a caliper.

**[TABLE 6]**

| **Treat concentration** | **Treat number** | **Number of surviving mice** |
|---|---|---|
| Atelocollagen-[AS1411-Dox Adduct] Complex AS1411-Dox Adduct 2 mg & 0.5% Atelocollagen | 3 | 3 |
| Atelocollagen-[CRO-Dox Adduct] Complex CRO-Dox Adduct 2 mg & 0.5% Atelocollagen | 3 | 3 |
| DPBS 0.5% Atelocollagen | 3 | 3 |

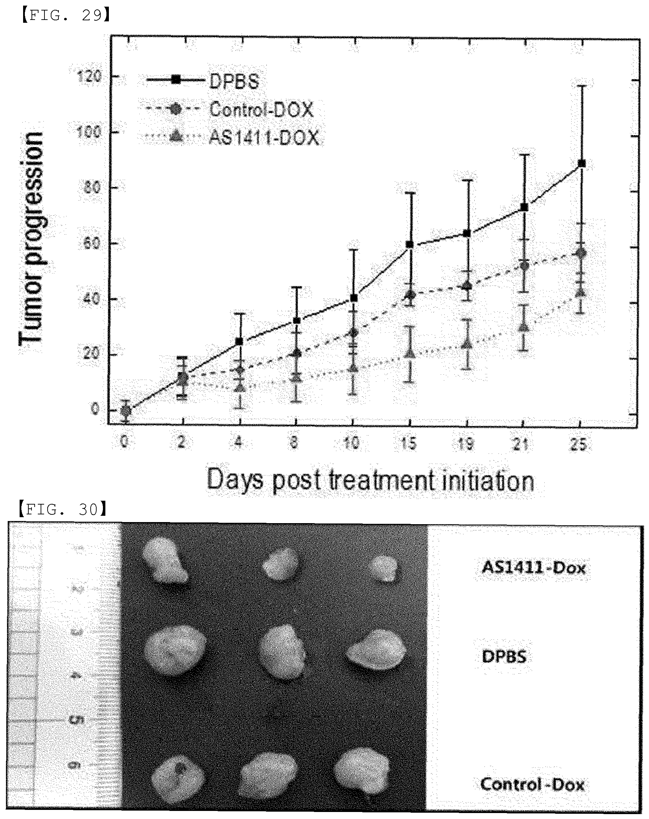

Efficacy validation of atelocollagen-[AS1411-Dox adduct] complex (sol-gel type): After the tumor has grown to an appropriate size, atelocollagen-[AS1411-Dox adduct] complex (sol-gel type) as shown in Table 6 above, was directly injected to a site of the grown tumor in each mouse through a syringe. Further, in order to determine anticancer effects of the complex, a change in the tumor size and a change in the weight of the implant were observed over a predetermined period of time. As compared to the control group, that is, a case of injecting 0.5% atelocollagen alone, as well as atelocollagen-[CRO-Dox adduct] complex (sol-gel type) without activity, it was observed that the atelocollagen-[AS1411-Dox adduct] complex (sol-gel type) has prominent cancer-inhibitory efficacy (FIG. 29).

At a time of 30 days passed after the transplantation of the atelocollagen-[AS11-Dox adduct] complex (sol-gel type), the tumors were excited from each group. All excited tumors were subjected to photographing, followed by comparing the actual tumor sizes to one another (FIG. 30).

### [Industrial Applicability]

The complex of the present invention is stable in a body and applicable to anticancer therapeutic agents and anti-inflammatory therapeutic agents.

## Claims

1. A complex for drug delivery and stabilization, comprising an atelocollagen-[aptamer-drug] complex.

2. The complex according to claim 1, wherein the drug is a substance with anticancer activity or anti-inflammatory activity.

3. The complex according to claim 1 or 2, wherein the drug is one or more selected from the group consisting of drugs, toxins, oligonucleotide drugs, antibodies, enzymes and nanoparticles.

4. A drug formulation comprising the complex according to claim 1.

5. The formulation according to claim 4, wherein the formulation is a topical formulation.

6. The formulation according to claim 5, wherein the topical formulation has a sol-gel, patch or scaffold form.

7. A method for stabilization of an aptamer-drug conjugate, comprising: conjugating a drug to an aptamer; and combining the conjugated product with atelocollagen to form a complex.
